# EUROPEAN PATENT APPLICATION

(11) **EP 4 455 267 A1**
(43) Date of publication of application: **30.10.2024**
(21) Application number: 22924589.9
(22) Date of filing: 02.12.2022
(51) Int. Cl.: C12N 5/07, C12N 5/10, C12M 1/00

(54) **CELL CULTURE METHOD AND MASKING SHEET**

(30) Priority: 03.02.2022 JP 2022015679
(71) Applicant: FUJIFILM Corporation, Tokyo 106-8620 (JP)
(72) Inventor: KASAI, Yusuke, Ashigarakami-gun, Kanagawa 258-8577 (JP); OBA, Takahiro, Ashigarakami-gun, Kanagawa 258-8577 (JP)
(74) Representative: Hoffmann Eitle
(86) International application number: PCT/JP2022/044596
(87) International publication number: WO 2023/149068

(57) **Abstract**

There is provided a masking sheet that is used for patterning cells or cell aggregates, which are formed on a culture surface. The masking sheet has the adhesive layer that is adhered to a culture surface, the support layer that is laminated on the adhesive layer to support the adhesive layer, and the opening portion that penetrates through the adhesive layer and the support layer and corresponds to a pattern of the cells or cell aggregates.

## Description

### BACKGROUND

### Technical Field

The disclosed technology relates to a cell culture method and a masking sheet.

### Related Art

The following technology is known as a technology for subjecting cells cultured on a culture surface to patterning. For example, JP2003-527615A describes a method of patterning cells, including shielding a first portion of a surface of a product with a pressure-sensitive adhesive mask that comes into contact with the surface of the product, applying a cell adhesive onto a second portion of the surface of the product through a channel in the mask while preventing the application of the cell adhesive onto the first portion of the surface of the product, and applying cells onto the cell adhesive.

The patterned culture technology for subjecting cells adherently cultured on a culture surface to desired patterning makes it possible to compartmentalize cells or cell aggregates into a desired shape and size and carry out culture or evaluation for each compartmentalized unit of cells. The compartmentalization makes it possible to quantify or standardize a state of the cells in each compartment, which consequently makes it possible to contribute to the stability of the evaluation system. It is expected that the simple patterned culture technology will lead to the accelerated development of life science fields such as drug discovery and regenerative medicine, which handle adherent cells.

JP2003-527615A describes that a patterned culture is carried out using a pressure-sensitive adhesive mask. The pressure-sensitive adhesive mask described in this document is composed of a sufficiently flexible polymeric elastomer in order to improve adhesiveness to a culture surface. However, such a mask having high flexibility has a problem in handleability, which makes it difficult to handle in a work process of patterned culture. For example, in a case where the mask is attached to a substrate having a surface that is to be a culture surface, it is not easy to maintain the mask in a state without bending or wrinkling. Further, there is also a concern about the deformation of the pattern due to the expansion and contraction of the mask. As described above, the use of the mask having high flexibility is a factor that reduces the productivity in the patterned culture.

### SUMMARY

The disclosed technology has been made in consideration of the above points, and an object of the disclosed technology is to improve the productivity in the patterned culture by improving the handleability of the mask that is used for the patterned culture.

A cell culture method according to the disclosed technology includes attaching a masking sheet having an opening portion to a culture surface and forming cells or cell aggregates a portion of the culture surface, which is exposed in the opening portion. The masking sheet includes an adhesive layer that is adhered to the culture surface, and a support layer that is laminated on the adhesive layer to support the adhesive layer.

The cell culture method according to the disclosed technology may include a step of applying a cell adhesive onto the culture surface using the masking sheet as a mask, a step of seeding cells on the culture surface after applying the cell adhesive, and a step of peeling off the masking sheet from the culture surface after applying the cell adhesive and before seeding the cells, or after seeding the cells.

The masking sheet according to the disclosed technology is a masking sheet for patterning cells or cell aggregates, which are formed on a culture surface, where the masking sheet includes an adhesive layer that is adhered to the culture surface, a support layer that is laminated on the adhesive layer to support the adhesive layer, and an opening portion that penetrates through the adhesive layer and the support layer and corresponds to a pattern of the cells or the cell aggregates.

A Young's modulus of the support layer is preferably higher than a Young's modulus of the adhesive layer, and it is preferably 0.2 GPa or more and 200 GPa or less. The Young's modulus of the adhesive layer is preferably 1 MPa or more and 100 MPa or less.

A thickness of the support layer is preferably 10 µm or more and 1,000 µm or less, and a thickness of the adhesive layer is preferably 1 µm or more and 100 µm or less.

The masking sheet may have a plurality of opening portions that have the same size and are arranged to be spaced by a gap. The opening portion may be formed by laser processing.

According to the disclosed technology, the handleability of the mask that is used for the patterned culture is improved, and thus it is possible to improve the productivity in the patterned culture.

### BRIEF DESCRIPTION OF THE DRAWINGS

Fig. 1 is a perspective view showing one example of a configuration of a masking sheet according to an embodiment of the disclosed technology.
Fig. 2 is a cross-sectional view taken along a line 2-2 in Fig. 1.
Fig. 3A is a view showing one example of a cell culture method according to an embodiment of the disclosed technology.
Fig. 3B is a view showing one example of the cell culture method according to the embodiment of the disclosed technology.
Fig. 3C is a view showing one example of the cell culture method according to the embodiment of the disclosed technology.
Fig. 3D is a view showing one example of the cell culture method according to the embodiment of the disclosed technology.
Fig. 3E is a view showing one example of the cell culture method according to the embodiment of the disclosed technology.
Fig. 3F is a view showing one example of the cell culture method according to the embodiment of the disclosed technology.
Fig. 3G is a view showing one example of the cell culture method according to the embodiment of the disclosed technology.
Fig. 4 shows photomicrographic images showing a pattern of opening portions of the masking sheet according to Example of the disclosed technology.
Fig. 5 shows photomicrographic images of fluorescently stained vitronectin according to Example of the disclosed technology.
In Fig. 6, an upper part shows photomicrographic images showing a pattern of opening portions of the masking sheet, a middle part shows photomicrographic images showing a pattern of vitronectin, and a lower part shows photomicrographic images showing patterned iPS cells.
Fig. 7A shows an external image of a masking sheet according to another Example of the disclosed technology.
Fig. 7B is a photomicrographic image of patterned vitronectin according to another Example of the disclosed technology.
Fig. 7C is a photomicrographic image of patterned iPS cells according to another Example of the disclosed technology.

### DETAILED DESCRIPTION

Hereinafter, one example of the embodiment of the disclosed technology will be described with reference to the drawings. It is noted that the same or equivalent constitutional elements and portions in the drawings are assigned by the same reference numerals, and overlapping descriptions will be omitted.

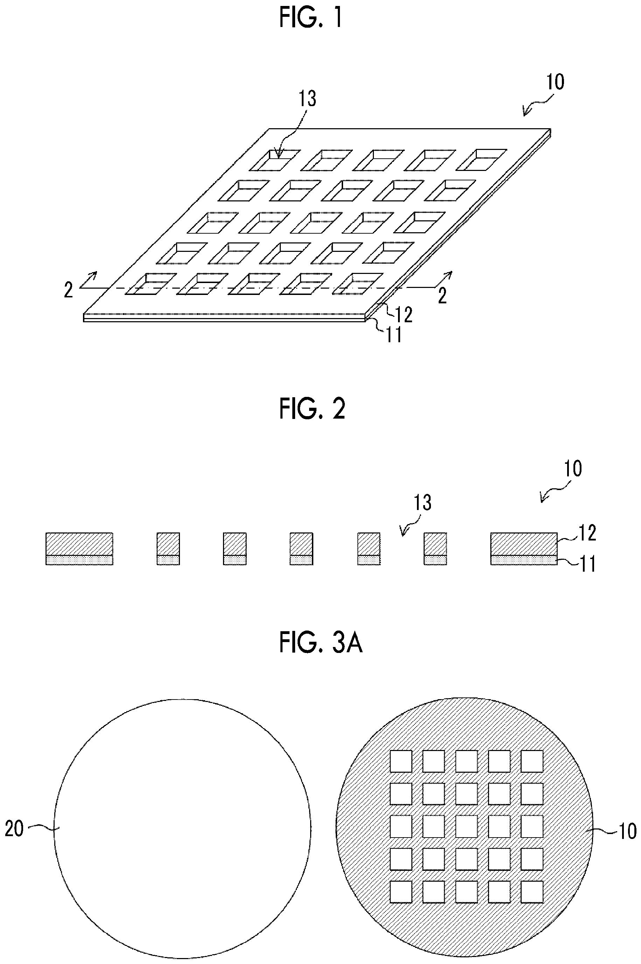

Fig. 1 is a perspective view showing one example of a configuration of a masking sheet 10 according to an embodiment of the disclosed technology. Fig. 2 is a cross-sectional view taken along the line 2-2 in Fig. 1. The masking sheet 10 is a sheet-shaped member that is used for patterning cells or cell aggregates, which are cultured on a culture surface.

The masking sheet 10 includes an adhesive layer 11 and a support layer 12. The adhesive layer 11 is a layer that is adhered to a surface (culture surface) of a substrate (not shown in the drawing) that is used for an adhesion culture of cells. The adhesive layer 11 preferably has sufficient flexibility in order to ensure the adhesiveness to the culture surface. Therefore, a Young's modulus of the adhesive layer 11 is preferably 1 MPa or more and 100 MPa or less. In addition, a thickness of the adhesive layer 11 is preferably 1 µm or more and 100 µm or less. In a case where the Young's modulus and the thickness of the adhesive layer 11 is set in the above-described ranges, it is possible to ensure the adhesiveness to the culture surface.

In addition, it is preferable that the adhesive layer 11 is formed from a material that has corrosion resistance to water, is not toxic to cells, and is suitable for thin film processing. As a material of the adhesive layer 11, for example, a rubber member such as silicone rubber (VMQ, PVMQ, or FVMQ), isoprene rubber (IR), or fluororubber (FKM) can be suitably used.

The support layer 12 is laminated on the adhesive layer 11. The support layer 12 has a function of supporting the adhesive layer 11 having high flexibility. Therefore, A Young's modulus of the support layer 12 is preferably higher than a Young's modulus of the adhesive layer 11, and it is preferably 0.2 GPa or more and 200 GPa or less. In addition, the thickness of the support layer 12 is preferably 10 µm or more and 1,000 µm or less. In a case where the Young's modulus and the thickness of the support layer 12 is set in the above-described ranges, it is possible to ensure the necessary rigidity for supporting the adhesive layer 11 in the support layer 12.

In addition, it is preferable that the support layer 12 is formed from a material that has corrosion resistance to water, is not toxic to cells, and is suitable for thin film processing. As a material of the support layer 12, for example, resin members such as polyimide, polyethylene terephthalate (PET), polycarbonate (PC), polypropylene (PP), polyvinyl chloride (PCV), polyvinyl alcohol (PVA), polyethylene (PE), polystyrene (PS), and hard rubber can be suitably used. In addition, as the material of the support layer 12, aluminum, stainless steel, copper, silver, gold, platinum, titanium, chromium, cobalt, nickel, glass, and ceramics can also be used.

The masking sheet 10 has a plurality of opening portions 13 that penetrate through the support layer 12 and the adhesive layer 11. The number, shape, and size of the opening portion 13, and the interval between the adjacent opening portions 13 can be freely determined. Cells or cell aggregates to be subjected to patterned culture are formed on the culture surface to have a pattern corresponding to the pattern of the opening portion 13 of the masking sheet 10. The masking sheet 10 may have a plurality of the opening portions 13 that have the same shape and the same size and are arranged to be spaced by a gap. According to such a pattern of the opening portion 13, it is possible to compartmentalize the cells or the cell aggregates and carry out culture or evaluation for each compartmentalized unit of cells. In addition, it is possible to quantify or standardize the state of the cells in each compartment.

Fig. 1 and Fig. 2 show an example of a configuration in which a plurality of the rectangular opening portions 13 having the same size are disposed in a matrix shape. The opening portion 13 can be formed by, for example, laser processing. That is, the opening portion 13 is formed by irradiating, with laser light, a sheet in which the adhesive layer 11 and the support layer 12 are laminated. According to the laser processing, it is possible to easily and accurately form the fine opening portion 13 that has a size having, for example, an order of magnitude of tens of micrometers. It is noted that the pattern of the opening portion 13 may be formed by cast molding.

Fig. 3A to Fig. 3G are views showing one example of a cell culture method in which a patterned culture is carried out using the masking sheet 10. First, a substrate 20 and the masking sheet 10 are prepared (Fig. 3A).

A surface of the substrate 20 constitutes a culture surface. A commercially available cell culture dish can be used as the substrate 20. The masking sheet 10 may be cut into the same shape as the shape of the substrate 20 (the circular shape in the example shown in Fig. 3A).

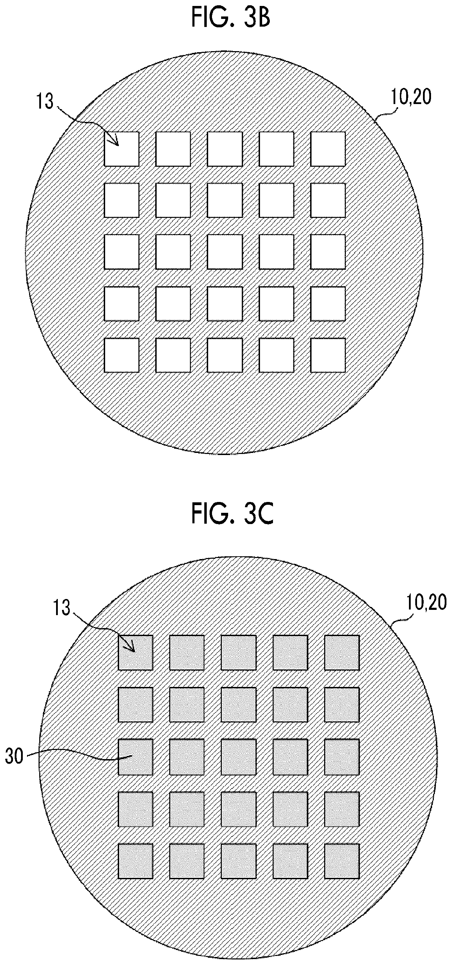

Next, the masking sheet 10 is attached to the surface (culture surface) of the substrate 20 (Fig. 3B). Specifically, the adhesive layer 11 of the masking sheet 10 is brought into contact with the surface (culture surface) of the substrate 20, and then a load is applied to the masking sheet 10 to closely attach the masking sheet 10 to the substrate 20. The flexibility of the adhesive layer 11 ensures high adhesiveness, and the masking sheet 10 is attached to the substrate 20 without generating air bubbles between the masking sheet 10 and the substrate 20. The surface (culture surface) of the substrate 20 is exposed in the opening portion 13 of the masking sheet 10. The diameter of the masking sheet 10 is preferably the same as the diameter of the substrate 20. As a result, the region in which a cell adhesive 30 adheres and the region in which a cell 40 adheres, other than the opening portion 13, are reduced.

Next, the cell adhesive 30 is applied onto the surface (culture surface) of the substrate 20 using the masking sheet 10 as a mask (Fig. 3C). As a result, the cell adhesive 30 adheres to a portion of the surface (culture surface) of the substrate 20, the portion being exposed in the opening portion 13 of the masking sheet 10. As a result, a pattern of the cell adhesive 30, which corresponds to the pattern of the opening portion 13 of the masking sheet 10, is formed on the culture surface. As the cell adhesive 30, it is possible to use an extracellular matrix protein such as vitronectin, laminin, fibronectin, collagens, or gelatins.

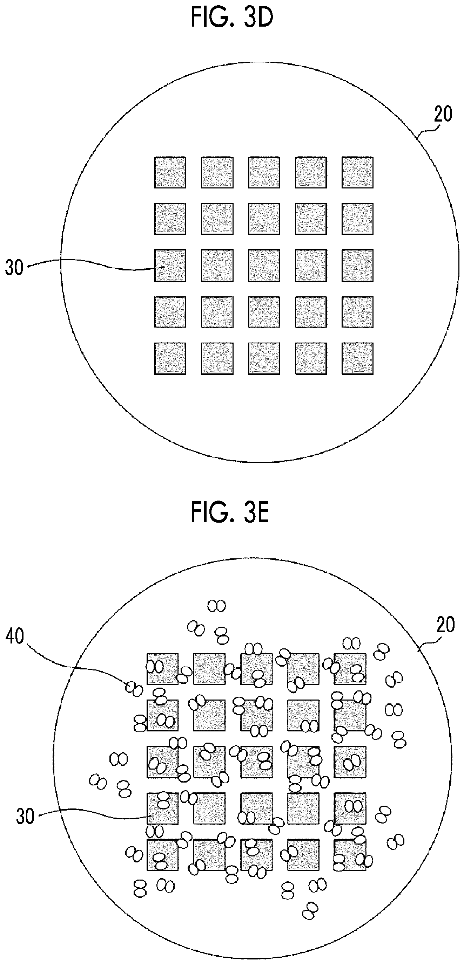

Next, the cell adhesive 30 is substituted with a phosphate buffer solution, and then the masking sheet 10 is peeled off from the culture surface (Fig. 3D). The surface (culture surface) of the substrate 20 maintains the patterned cell adhesive 30. Next, a blocking agent is applied onto the surface (culture surface) of the substrate 20. This treatment is a blocking treatment for inhibiting non-specific cell adhesion in a region other than the region in which the cell adhesive 30 has adhered to the culture surface. As the blocking agent, for example, a bovine serum albumin solution prepared at a predetermined concentration can be used.

Next, the cell 40 adjusted to a predetermined density is seeded on the surface (culture surface) of the substrate 20 (Fig. 3E). The cell 40 may be any adhesive cell, and it is possible to use, for example, stem cells such as induced pluripotent stem cells (iPS cell), an embryonic stem cell (ES cell), a mesenchymal stem cell, and a hematopoietic stem cell, somatic cells such as a lung cell, a stomach cell, a liver cell, a kidney cell, an intestinal cell, a nerve cell, an eye cell, a vascular endothelial cell, a myocardial cell, and a bone cell, cancer cells such as a lung cancer cell, a stomach cancer cell, a colon cancer cell, a breast cancer cell, a leiomyosarcoma cell, a fibrosarcoma cell, and an osteosarcoma cell, and established cell lines such as an HEK293 cell (human embryonic kidney cell) and a Vero cell (African green monkey kidney cell). Typically, an iPS cell derived from a human can be used.

In addition, a cell derived from an animal (specifically, a mouse, a rat, a rabbit, a pig, a dog, a monkey, a cow, a horse, a sheep, a chicken, and the like) other than a human may be used.

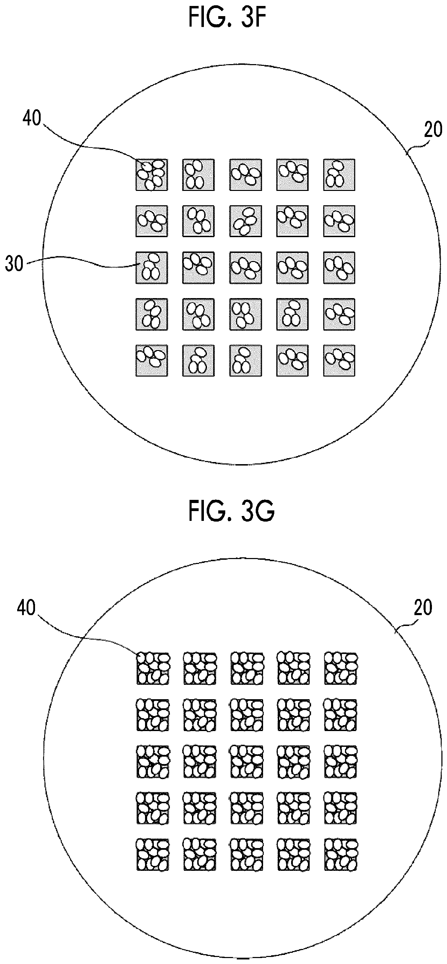

Thereafter, the static culture of the cell 40 is carried out. During the culture period, the substrate 20 on which the cells 40 are seeded is accommodated in an incubator that is maintained at an appropriate temperature and an appropriate CO₂ concentration. The cell 40 adheres in a region of the surface (culture surface) of the substrate 20, in which the cell adhesive 30 has adhered (Fig. 3F).

The culture is continued while carrying out the medium replacement at a predetermined timing. As a result, the cell 40 proliferates in a region of the surface (culture surface) of the substrate 20, in which the cell adhesive 30 has adhered. As a result, it is possible to obtain cells or cell aggregates (colonies), which have a pattern corresponding to the pattern of the opening portion 13 of the masking sheet 10 (Fig. 3G). It is noted that in the present embodiment, although a case where the masking sheet 10 is peeled off from the culture surface after applying the cell adhesive 30 and before seeding the cell 40 has been shown as an example, the masking sheet 10 may be peeled off from the culture surface after applying the cell adhesive 30 and after seeding the cell 40.

As described above, the masking sheet 10 according to the embodiment of the disclosed technology is used for patterning cells or cell aggregates, which are formed on a culture surface. The masking sheet 10 has the adhesive layer 11 that is adhered to a culture surface, the support layer 12 that is laminated on the adhesive layer 11 to support the adhesive layer 11, and the opening portion 13 that penetrates through the adhesive layer 11 and the support layer 12 and corresponds to a pattern of the cells or cell aggregates. In addition, the cell culture method according to the embodiment of the disclosed technology is a method of attaching a masking sheet 10 to a culture surface and forming cells or cell aggregates in the portion 13 exposed in the opening portion of the masking sheet 10 of the culture surface, where cell culture method includes a step of applying the cell adhesive 30 onto the culture surface using the masking sheet 10 as a mask, a step of seeding cells on the culture surface after applying the cell adhesive 30, and a step of peeling off the masking sheet 10 from the culture surface after applying the cell adhesive 30 and before seeding the cells, or after seeding the cells.

The masking sheet 10 has not only the adhesive layer 11 but also the support layer 12 that supports the adhesive layer 11. Since the support layer 12 is included in the masking sheet 10, rigidity is imparted to the masking sheet 10. As a result, the handleability is improved as compared with a masking sheet composed of only the adhesive layer 11 having high flexibility, which makes it possible to facilitate handling in the work process of patterned culture. For example, in a case where the masking sheet 10 is attached to the substrate 20, it is easy to maintain the masking sheet 10 in a state without bending or wrinkling. Further, it is possible to suppress the deformation of the pattern due to the expansion and contraction of the masking sheet 10. That is, according to the masking sheet 10 and the cell culture method using the masking sheet 10 according to the embodiment of the disclosed technology, the handleability of the mask that is used for the patterned culture is improved, and thus it is possible to improve the productivity in the patterned culture.

### [Examples]

Hereinafter, the disclosed technology will be described in more detail with reference to Examples. However, the disclosed technology is not limited to the following Examples.

### <Preparation of masking sheet>

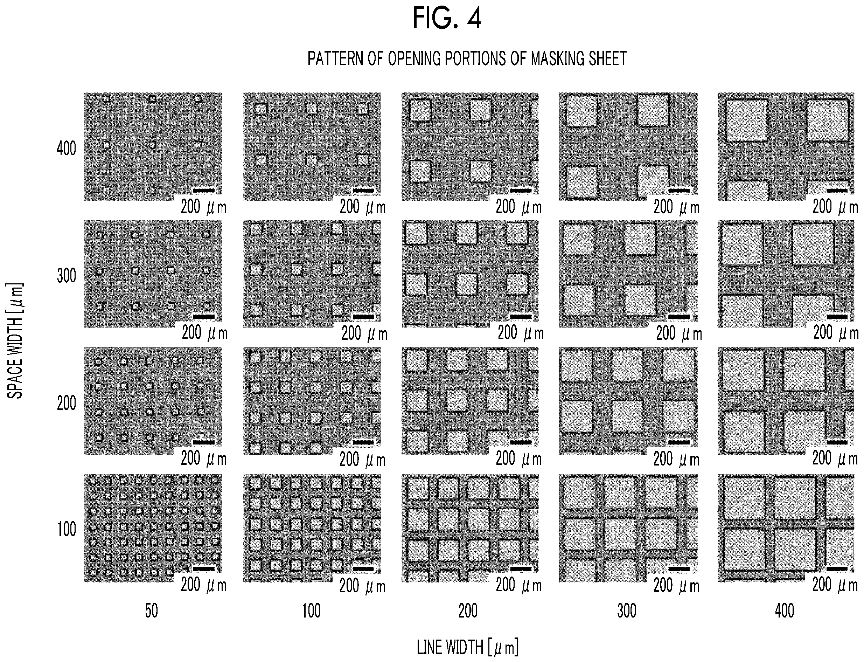

A masking sheet, in which a pressure-sensitive adhesive layer was formed from a silicone-based pressure-sensitive adhesive and a support layer was formed from Kapton (registered trademark), which is one kind of a polyimide film, was prepared. The opening portion of the masking sheet was formed by laser processing. Twenty kinds of the masking sheets 10 which have combinations of the line width of the opening portion (50 µm, 100 µm, 200 µm, 300 µm, or 400 µm) and the space width of the opening portion (100 µm, 200 µm, 300 µm, or 400 µm), different from each other, were prepared (see Fig. 4). All of the shapes of the opening portions are square. It is noted that the line width is a length of one side of the opening portion, and the space width is an interval between the opening portions adjacent to each other.

### <Patterned culture>

The masking sheet was attached to a surface of a commercially available 35 mm dish (manufactured by Falcon). Specifically, the adhesive layer of the masking sheet was brought into contact with the surface of the dish, and then a load was applied to the masking sheet to closely attach the masking sheet to the surface of the dish.

Next, a vitronectin solution having a concentration of 2.5 ug/ml, which was a cell adhesive, was applied onto the surface of the dish using the masking sheet as a mask, and then the dish was allowed to stand for 1 hour. As a result, a pattern of the cell adhesive, corresponding to the pattern of the opening portion of the masking sheet was formed on the surface of the dish.

Next, the vitronectin solution was substituted with a phosphate buffer solution, and then the masking sheet was peeled off from the surface of the dish.

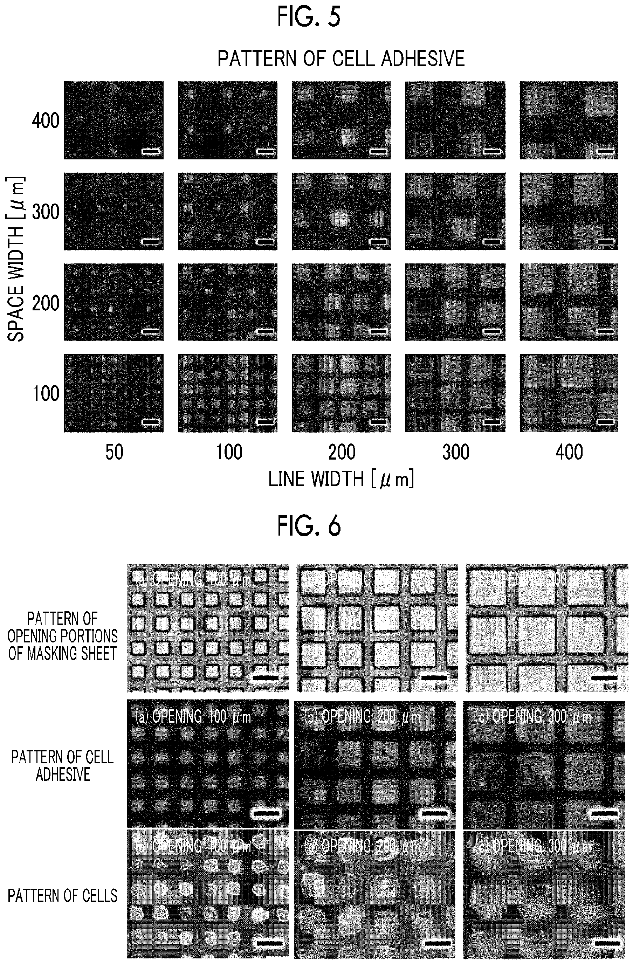

Next, a 3% bovine serum albumin solution, which was a blocking agent, was applied onto the surface of the dish. This treatment is a blocking treatment for inhibiting non-specific cell adhesion in a region other than the region in which the cell adhesive has adhered to the culture surface. The treatment time was set to 30 minutes. Thereafter, the blocking agent was substituted with a phosphate buffer solution, and the phosphate buffer solution was aspirated. At this stage, the fluorescence of vitronectin was observed in order to confirm that the pattern of vitronectin, which is a cell adhesive, is maintained on the surface of the dish. Fig. 5 shows photomicrographic images of the fluorescently stained vitronectin. As shown in Fig. 5, it was confirmed that regarding all of the twenty kinds of patterns described above, the pattern of vitronectin is maintained on the surface of the dish.

Next, a cell suspension of human-derived iPS cells having a concentration of 0.1 M cells/mL was seeded in a dish, and static culture was carried out for 24 hours. During the culture period, the dish was accommodated in an incubator maintained at 37°C and a CO₂ concentration of 5%. Thereafter, the culture medium was repalced to remove floating cells. As a result, a pattern of cells was formed on the surface of the dish.

The lower part of Fig. 6 shows photomicrographic images of the iPS cells patterned by the patterned culture using masking sheets in which the line width of the opening portion is 100 µm, 200 µm, and 300 µm, respectively, and the space width is 100 µm. The upper part of Fig. 6 shows the corresponding photomicrographic images showing a pattern of opening portions of the masking sheet, and the middle part of Fig. 6 shows the corresponding photomicrographic images showing a pattern of vitronectin. As shown in Fig. 6, it was confirmed that a cell pattern corresponding to the pattern of the opening portion of the masking sheet is formed.

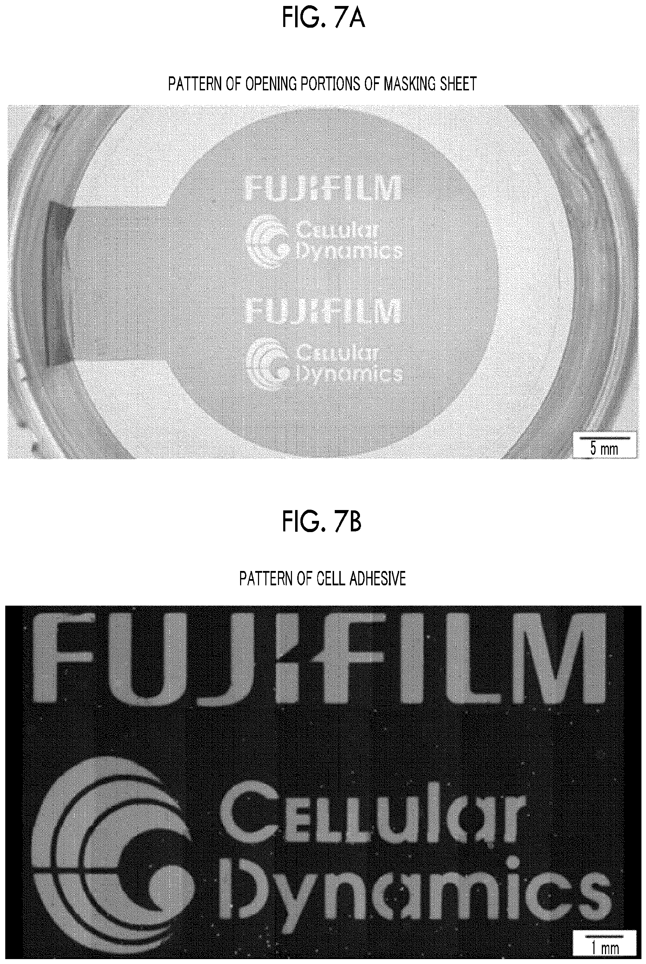

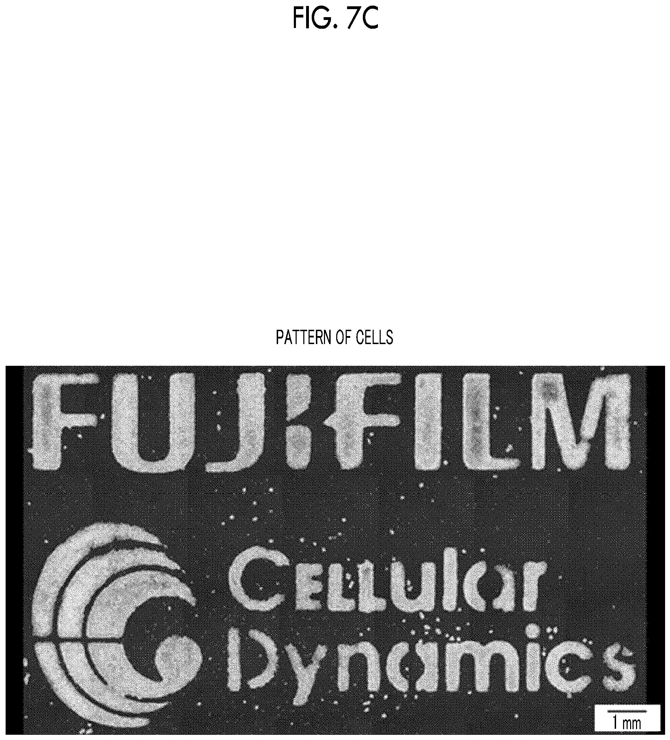

Fig. 7A shows an external image of a masking sheet according to another Example of the disclosed technology. Fig. 7B is a photomicrographic image of vitronectin, which is a cell adhesive patterned using the masking sheet shown in Fig. 7A. Fig. 7C is a photomicrographic image of the iPS cells patterned using the masking sheet shown in Fig. 7A. As shown in Fig. 7A to Fig. 7C, according to the disclosed technology, it is possible to realize the formation of a more complicated cell pattern with high productivity.

The disclosure of JP2022-015679 is incorporated in the present specification in its entirety by reference.

All documents, patent applications, and technical standards described in the present specification are incorporated herein by reference, to the same extent as in the case where each of the documents, patent applications, and technical standards is specifically and individually described.

## Claims

1. A cell culture method for attaching a masking sheet having an opening portion to a culture surface and forming cells or cell aggregates a portion of the culture surface, which is exposed in the opening portion,
wherein the masking sheet includes an adhesive layer that is adhered to the culture surface, and a support layer that is laminated on the adhesive layer to support the adhesive layer.

2. The cell culture method according to claim 1,
wherein a Young's modulus of the support layer is higher than a Young's modulus of the adhesive layer.

3. The cell culture method according to claim 1 or 2,
wherein a Young's modulus of the support layer is 0.2 GPa or more and 200 GPa or less.

4. The cell culture method according to any one of claims 1 to 3,
wherein a Young's modulus of the adhesive layer is 1 MPa or more and 100 MPa or less.

5. The cell culture method according to any one of claims 1 to 4,
wherein a thickness of the support layer is 10 µm or more and 1,000 µm or less.

6. The cell culture method according to any one of claims 1 to 4,
wherein a thickness of the adhesive layer is 1 µm or more and 100 µm or less.

7. The cell culture method according to any one of claims 1 to 6,
wherein the masking sheet has a plurality of opening portions that have the same size and are arranged to be spaced by a gap.

8. The cell culture method according to any one of claims 1 to 7,
wherein the opening portion is formed by laser processing.

9. The cell culture method according to any one of claims 1 to 8, comprising:
a step of applying a cell adhesive onto the culture surface using the masking sheet as a mask;
a step of seeding cells on the culture surface after applying the cell adhesive; and
a step of peeling off the masking sheet from the culture surface after applying the cell adhesive and before seeding the cells, or after seeding the cells.

10. A masking sheet for patterning cells or cell aggregates, which are formed on a culture surface, the masking sheet comprising:
an adhesive layer that is adhered to the culture surface;
a support layer that is laminated on the adhesive layer to support the adhesive layer; and
an opening portion that penetrates through the adhesive layer and the support layer and corresponds to a pattern of the cells or the cell aggregates.

11. The masking sheet according to claim 10,
wherein a Young's modulus of the support layer is higher than a Young's modulus of the adhesive layer.

12. The masking sheet according to claim 10 or 11,
wherein a Young's modulus of the support layer is 0.2 GPa or more and 200 GPa or less.

13. The masking sheet according to any one of claims 10 to 12,
wherein a Young's modulus of the adhesive layer is 1 MPa or more and 100 MPa or less.

14. The masking sheet according to any one of claims 10 to 13,
wherein a thickness of the support layer is 10 µm or more and 1,000 µm or less.

15. The masking sheet according to any one of claims 10 to 14,
wherein a thickness of the adhesive layer is 1 µm or more and 100 µm or less.

16. The masking sheet according to any one of claims 10 to 15,
wherein the masking sheet has a plurality of opening portions that have the same size and are arranged to be spaced by a gap.

17. The masking sheet according to any one of claims 10 to 16,
wherein the opening portion is formed by laser processing.
